(19) Europäisches Patentamt
European Patent Office
Office européen des brevets

(11) Numéro de publication : **0 549 418 A2**

# (12) DEMANDE DE BREVET EUROPEEN

(21) Numéro de dépôt : **92403435.8**

(22) Date de dépôt : **17.12.92**

(51) Int. Cl.[5] : **C07C 233/10,** C07C 233/11, A01N 37/18

(30) Priorité : **23.12.91 US 811439**

(43) Date de publication de la demande : **30.06.93 Bulletin 93/26**

(84) Etats contractants désignés : **AT BE CH DE DK ES FR GB GR IE IT LI LU NL PT SE**

(71) Demandeur : **ROUSSEL-UCLAF**
**35, Boulevard des Invalides**
**F-75007 Paris (FR)**

(72) Inventeur : **Blade, Robert John**
**23 Fantail Lane**
**Tring, Herts HP23 4EN (GB)**
Inventeur : **Cockerill, George Stuart**
**33 Hartwort Close, Walnut Tree**
**Milton Keynes MK7 7LJ (GB)**

(74) Mandataire : **Niel, Michel et al**
**ROUSSEL-UCLAF Boîte postale no 9 111,**
**route de Noisy**
**F-93230 Romainville (FR)**

## (54) Amides insaturés ayant une activité pesticide.

(57) La présente invention décrit des composés actifs du point de vue pesticide, de formule (I)

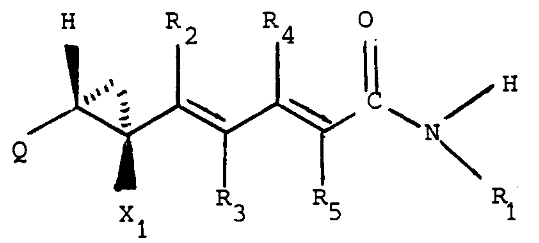

(I)

ou un sel de celui-ci, dans lequel Q est un noyau aromatique monocyclique ou un système de noyau bicyclique fusionné dont au moins un cycle est aromatique contenant 9 ou 10 atomes dont l'un peut être l'azote et les autres le carbone, chaque cycle étant éventuellement substitué, ou Q est un groupement dihalo-génovinyle, ou un groupement $R_6$-C≡C- dans lequel $R_6$ est un alkyle en $C_{1-4}$ tri(alkyle en $C_{1-4}$)silyle, halogène ou hydrogène ; $R_2$, $R_3$, $R_4$ et $R_5$ sont identiques ou différents, l'un au moins étant hydrogène et les autres étant choisis indépendamment parmi hydrogène, halogéno, alkyle en $C_{1-4}$ Ou halogénoalkyle en $C_{1-4}$ ; et $R_1$ est choisi parmi hydrogène et hydrocarbyle en $C_{1-8}$ éventuellement substitué par dioxalanyle, halogéno, cyano, trifluorométhyle, trifluorométhylthio ou alkoxy en $C_{1-6}$ et $X_1$ est hydrogène, fluoro ou chloro.

EP 0 549 418 A2

Cette demande est une continuation en partie de l'USSN 729847 déposée le 7 décembre 1991.

Cette invention concerne des composés pesticides, leurs procédés de préparation, des compositions les contenant et leur utilisation pour la lutte contre des organismes nuisibles.

Des amides insaturés ayant une chaîne méthylénique de 1 à au moins 10 atomes de carbone comportant éventuellement au moins un oxygène ou un groupement méthylénique supplémentaire sont connus à titre de pesticides ou d'insecticides ayant divers groupement terminaux qui comportent parmi eux un phényle éventuellement substitué (demande européenne Nos. 228222, 194764, 225011, demande japonaise Nos. 57-212150, Meisters et Wailes: Aust. J. Chem. 1966, 19, 1215, Vig et al: J. Ind. Chem. Soc. 1974, 51(9), 817) ou un pyridyle (demande européenne 269457) ou un système de noyau bicyclique fusionné (demande européenne Nos. 143593, 228853), un dihalogénovinyle ou un éthynyle éventuellement substitué (demande européenne 228222).

Aucun groupement interstitiel cycloalkyle liant le motif diène au groupement terminal n'y est décrit.

H.O. Huisman et al, Rev. trav. chim. 77, 97-102, (1958) décrit un groupement de 5-(2,6,6-triméthylcyclohexényl)-2,4-pentadiénamides comme insecticides.

La demande de brevet européen 369762A (demande de brevet américain SN 07/436803) a pour objet un composé de formule (I):

$$Q\ Q_1\ CR_2 = CR_3\ CR_4 = CR_5C\ (=X)\ NHR_1$$

ou un sel de celui-ci, dans lequel Q est un noyau aromatique monocyclique ou un système de noyau bicyclique fusionné dont au moins un cycle est aromatique contenant 9 ou 10 atomes dont l'un des atomes peut être un azote et les autresdes atomes de carbone, chaque cycle étant éventuellement substitué, ou Q est un groupement dihalogénovinyle, ou un groupement $R_6$-C≡C- dans lequel $R_6$ est un radical alkyle en $C_{1-4}$, tri(alkyle en $C_{1-4}$)silyle ou un atome d'halogène ou d'hydrogène; $Q_1$ est un noyau 1,2-cyclopropyle éventuellement substitué par un ou plusieurs groupement choisis parmi un alkyle en $C_{1-3}$, halogéno, halogénoalkyle en $C_{1-3}$, alkynyle ou cyano; $R_2$, $R_3$, $R_4$ et $R_5$ sont identiques ou différents, l'un au moins étant un atome d'hydrogène et les autres étant choisis indépendamment parmi les atomes d'hydrogène, d'halogène, les radicaux alkyle en $C_{1-4}$ ou halogénoalkyle en $C_{1-4}$; X est oxygène ou soufre; et $R_1$ est un atome d'hydrogène ou un radical hydrocarboné en $C_{1-8}$ éventuellement substitué par dioxalanyle, halogéno, cyano, trifluorométhyle, trifluorométhylthio ou alkoxy en $C_{1-6}$ et $X_1$ est est un atome d'hydrogène, de fluor ou de chlore.

On a maintenant trouvé que certains énantiomères des composés de formule (I) ont, de manière étonnante, une forte activité insecticide par rapport aux autres isomères.

De ce fait, la présente invention a pour objet un composé de formule (I):

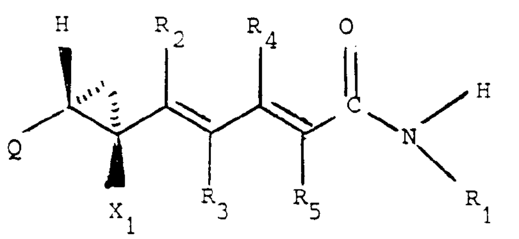

(I)

dans lequel Q et $R_1$ à $R_5$ sont tels que définis précédemment et $X_1$ est hydrogène ou fluoro ou chloro.

Lorsque Q est un noyau aromatique monocyclique, celui-ci peut être phényle, pyridyle, ou thiényle et de préférence phényle. Lorsque Q est un système de noyau bicyclique, celui-ci est de préférence un naphtyle.

Lorsque Q contient un système aromatique substitué, on entend 1 à 4 substituants identiques ou différents choisis parmi radical hydrocarboné en $C_{1-6}$, alkoxy en $C_{1-6}$ méthylènedioxy, chacun étant éventuellement substitué par un à trois halogénes ou encore parmi halogèno, cyano ou nitro, ou le substituant est un groupement $S(O)_nR_7$ dans lequel n est 0, 1 ou 2 et $R_7$ est alkyle en $C_{1-6}$ éventuellement substitué par un ou plusieurs halogéno ou $R_7$ est amino éventuellement substitué par un ou deux groupements alkyle en $C_{1-6}$, ou le substituant est un groupement $NR_6R_9$ où $R_8$ et $R_9$ sont indépendamment choisis parmi hydrogène, alkyle en $C_{1-6}$ ou le substituant est un groupement $COR_{10}$ où $R_{10}$ est alkyle en $C_{1-6}$.

Le noyau Q contient normalement jusqu'à trois substituants et peut être non substitué ou substitué par un, deux ou trois substituants tels que halogéno ou halogénoalkyle en $C_{1-4}$ tel que trifluorométhyle. La substitution du noyau Q dépend de la nature de ce noyau mais elle est de préférence en position 3, 4 ou 5 lorsque Q est un noyau à 6 sommets.

$R_2$, $R_3$, $R_4$ et $R_5$ sont choisis notamment parmi hydrogène, méthyle ou fluoro. La stéréochimie des doubles liaisons est notamment (E). Lorsque $R_3$ ou $R_6$ est fluoro, alors la stéréochimie de la double liaison à laquelle

$R_3$ ou $R_5$ est liée est notamment (Z). De préférence, $R_2$ est hydrogène, $R_3$ est hydrogène ou fluoro, $R_5$ est hydrogène ou fluoro et $R_4$ est hydrogène ou alkyle en $C_{1-4}$, très préférentiellement méthyle.

$R_1$ est particulièrement alkyle éventuellement substitué par cycloalkyle, dioxalanyle, ou $R_1$ est alkényle en $C_{2-5}$. $R_1$ est plus particulièrement un groupement alkyle ramifié en $C_{4-6}$ tel que isobutyle, 1,2-diméthylpropyle, 1,1,2-triméthylpropyle, 2,2-diméthylpropyle ou $R_1$ est 2-méthylprop-2-ényle ou (2-méthyl-1,3-dioxalane-2-yl)méthyle. De préférence, $R_1$ est isobutyle ou 2-méthyl-prop-2-ényle où $R_2$ et $R_3$ sont hydrogène, $R_4$ est méthyle et $R_5$ est hydrogène ou méthyl.

De préférence, la configuration stéréométrique du groupement cyclopropyle dans la chaîne est telle que le groupement Q et la chaîne latérale carbonée sont liés au noyau pour donner la géométrie trans.

Lorsque $X_1$ est fluoro ou chloro, la configuration absolue au niveau du noyau cyclopropane est de préférence (1R,2S). Lorsque $X_1$ est hydrogène, la configuration absolue au niveau du noyau cyclopropane est de préférence (1S,2R).

Parmi les composés de formule (I), l'invention a particulièrement pour objet les composés de formule (II):

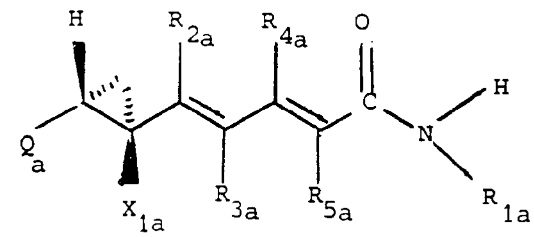

(II)

ou un sel de ceux-ci, dans lequel $Q_a$ est un groupement phényle ou pyridyle substitué; $X_{1a}$ est hydrogène, fluoro ou chloro; $R_{2a}$, $R_{3a}$, $R_{4a}$ et $R_{5a}$ sont identiques ou différents, l'un au moins étant hydrogène et les autres étant choisis indépendamment parmi hydrogène, halogéno, alkyle en $C_{1-4}$ ou halogénoalkyle en $C_{1-4}$ et $R_{1a}$ est choisi parmi hydrogène et hydrocarbyle en $C_{1-6}$ éventuellement substitué par dioxalanyle, halogéno, cyano, trifluorométhyle, trifluorométhylthio ou alkoxy en $C_{1-6}$.

Parmi les substituants appropriés de $Q_a$ on peut citer un ou plusieurs groupements choisis parmi halogéno, cyano, nitro, alkyle en $C_{1-6}$, alkoxy et méthylènedioxy, ceux-ci étant éventuellement substitués par un à cinq halogéno, ou encore le groupement $S(O)_nR_{7a}$ dans lequel n est 0, 1 ou 2 et $R_{7a}$ est alkyle en $C_{1-6}$ éventuellement substitué par halogéno, ou $R_{7a}$ est amino.

$R_{2a}$, $R_{3a}$, $R_{4a}$ et $R_{5a}$ sont choisis notamment parmi hydrogène, méthyle ou fluoro.

$R_{1a}$ est particulièrement alkyle en $C_{1-6}$ éventuellement substitué par dioxalanyle, ou $R_{1a}$ est alkényle en $C_{2-5}$. Plus particulièrement $R_{1a}$ est un groupement alkyle ramifié en $C_{4-6}$ tel que isobutyle, 1,2-diméthylpropyle, 1,1,2-triméthylpropyle, 2,2-diméthylpropyle ou $R_{1a}$ est 2-méthylprop-2-ényle ou (2-méthyl-1,3-dioxalane-2-yl)méthyle. De préférence, $R_{1a}$ est isobutyle ou 2-méthylprop-2-ényl où $R_{2a}$ et $R_{3a}$ sont hydrogène et $R_{4a}$ est méthyle.

Parmi les composés de formule (II) préférés, on peut citer ceux dans lesquels $R_{2a}$, $R_{3a}$ et $R_{5a}$ sont chacun hydrogène.

L'invention a plus particulièrement pour objet les composés de formule (II) dans lesquels $R_2$ est hydrogène.

L'invention a également particulièrement pour objet ceux dans lesquels Q est un phényle substitué, $R_4$ est méthyle ou hydrogène, $R_2$ est hydrogène, $R_3$ et $R_5$ sont hydrogène ou fluoro et $R_1$ est isobutyle ou 1,2-diméthylpropyle ou 2-méthylprop-2-ényl et X est oxygène ou soufre.

Ainsi, parmi les composés préférés de formule (I) on peut citer:

(c désigne le substituant cis, par rapport (r) à un autre substituant)

le (-)-(2E,4E)-N-(2-méthylprop-2-ényl)-5-[(1R,2S)-c-2-(3,4-dibromophényl)-r-1-fluorocyclopropyl]-3-méthylpenta-2,4-diénamide

le (-)-(2E/Z,4E)-N-(2-méthylprop-2-ényl)-5-[1R,2S)-c-2-(3,4-dibromophényl)-r-1-fluorocyclopropyl]-3-méthylpenta-2,4-diénamide

le (+)-(2E,4E)-N-(2-méthylprop-2-ényl)-5-[(1S,2R)-c-2-(3,4-dibromophényl)-r-1-fluorocyclopropyl]-3-méthylpenta-2,4-diénamide

le (+)-(2E/Z,4E)-N-(2-méthylprop-2-ényl)-5-[(1S,2R)-c-2-(3,4-dibromophényl)-r-1-fluorocyclopropyl]-3-méthylpenta-2,4-diénamide

le (+)-(2E,4E)-N-isobutyl-5-[(1S,2R)-c-(3,4-dichloro-phényl)-r-1-chlorocyclopropyl]-3-méthylpenta-2,4-diénamide

le (-)-(2E,4E)-N-isobutyl-5-[(1R,2S)-c-(3,4-dichlorophényl)-r-1-chlorocyclopropyl]-3-méthylpenta-2,4-diénamide

le (-)-(2E,4E)-N-(S-1-méthylpropyl)-5-[(1R,2S)-c-(3,4-dichlorophényl)-r-1-chlorocyclopropyl]-3-méthylpenta-2,4-diénamide

le (-)-(2E,4E)-N-(R-1-méthylpropyl)-5-[(1R,2S)-c-(3,4-dichlorophényl)-r-1-)chlorocyclopropyl]-3-méthylpenta-2,4-diénamide

le (-)-(2E/Z,4E)-N-isobutyl-5-[(1S,2R)-trans-2-(3,4-dibromophényl)cyclopropyl]-3-méthylpenta-2,4-diénamide

Le (+)-(2E/Z,4E)-N-isobutyl-5-[(1R,2S)-trans-2-(3,4-dibromophényl)cyclopropyl-3-méthylpenta-2,4-diénamide

Par l'expression halogéno, on entend fluoro, chloro, bromo et iodo.

Par l'expression radical hydrocarboné, on entend alkyle, alkényle, alkynyle, aralkyle comprenant un groupement alkyle ou alkényl cyclique éventuellement substitué par alkyle, alkényle ou alkynyle; et alkyle ou alkényle substitué par des groupements alkyles et alkényles cycliques ainsi que les groupements phényles.

Les sels des composés de la présente invention seront normalement des sels d'addition acide. De tels sels peuvent se former à partir d'acides minéraux ou organiques. Les sels préférés incluent ceux formés à partir des acides chlorhydrique, bromhydrique, sulfurique, citrique, nitrique, tartrique, phosphorique, lactique, benzoïque, glutamique, aspartique, pyruvique, acétique, succinique, fumarique, maléique, oxaloacétique, hydroxynaphtoïque, iséthionique, stéarique, méthanesulfonique, éthanesulfonique, benzènesulfonique, p-toluènesulfonique, lactobionique, glucuronique, thiocyanique, propionique, embionique, naphténoïque et perchlorique.

Selon un autre aspect, la présente invention a également pour objet un procédé de préparation d'un composé de formule (I ) tel que défini précédemment comprenant:

a) une réaction d'oxydation d'un alcool approprié de formule (III):

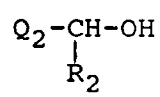

dans laquelle $R_2$ est tel que défini précédemment pour obtenir l'aldéhyde correspondant de formule ($III_A$):

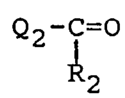

b) que l'on soumet, par une réaction de type Wittig à un réactif capable d'introduire le groupement

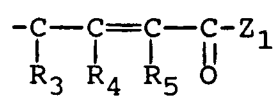

dans lequel $R_3$, $R_4$, $R_5$ et $Z_1$ sont tels que définis précédemment puis à l'action d'une amine de formule $H_2NR_1$, dans laquelle $R_1$ est tel que défini précédemment et $Z_1$ est hydroxy, alkoxy en $C_{1-6}$, halogéno ou un ester de phosphoroimidate (-P(O)(O-aryl)NH- aryle où aryle est un aryle en $C_{6-10}$)

et ensuite transformation éventuelle d'un composé de formule (I) en un autre composé de formule (I) par des méthodes bien connues de l'homme de l'art.

Le procédé (a) est habituellement effectué à une température qui n'est pas trop élevée, par exemple comprise entre -25 et 150°C dans un solvant aprotique anhydre, tel que l'éther, le dichlorométhane, le toluène ou le benzène. Les conditions précises seront fonction de la nature du groupement $Z^1$; par exemple, lorsque $Z^1$ est alkoxy, la réaction est commodément effectuée à une température élevée, c'est-à-dire de 50 à 125°C, et commodément sous reflux, de préférence en présence d'un composé trialkylaluminium tel que le triméthylaluminium, qui forme un complexe avec l'amine $H_2NR^1$. Lorsque $Z^1$ est halogéno ou phosphoroimidate, la réaction est commodément effectuée entre 0° et 30°C et notamment à température ambiante, de préférence en présence d'une amine tertiaire, telle que la triéthylamine.

Si le dérivé acide est un halogénure d'acide, par exemple le chlorure d'acide, alors il peut être formé à partir de l'acide correspondant par réaction avec un réactif approprié tel que le chorure d'oxalyle ou le chlorure de thionyle. Lorsque $Z^1$ est un groupement phosphoroimidate, alors celui-ci peut être convenablement formé à partir de (PhO)P(=O)NHPhCl où Ph est phényle. L'acide ou la fonction acide dans le composé $Q_2CR_2{=}CR_3CR_4{=}CR_5COZ_1$, peut être préparé par hydrolyse de l'ester correspondant dans lequel $Q_2$ représente le groupement:

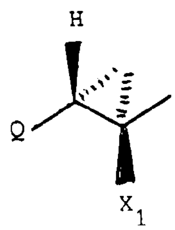

Les esters peuvent être préparés par plusieurs voies possibles, par exemple:

(i) réaction classique de Wittig ou de Wadsworth-Emmons à l'aide par exemple d'un aldéhyde et d'éthoxycarbonylméthylène-triphénylphosphorane ou un anion à partir de triéthylphosphonocrotonate ou 3-méthyl-triéthylphosphonocrotonate. Cette dernière réaction peut conduire à un mélange isomère, par exemple un mélange de diénoates substitués (Z) et (E); on peut faire réagir un tel mélange comme précédemment et le mélange d'amides résultant est séparé par chromatographie ou autres techniques appropriées.

Le réactif de Wittig peut être préparé par exemple par la voie suivante ou une modification de celle-ci:

$$(CH_3)_2C{=}CHCO2Et \xrightarrow[(2)]{(1)} Z_2CH_2C(CH_3){=}CHCO_2Et \xrightarrow{(3)} \text{réactif de Wittig/ Wadsworth-Emmons}$$

dans laquelle $Z_2$ = $(aryl)_3P$, $(aryl)_2P(0O$ ou $(C_{1-4}\ alkoxy)_2P(O)$ où aryle est de préférence phényle, et alkoxy est de préférence éthoxy.

(1) N- bromo succinimide

(2) par exemple, $(EtO)_3$ ou $(Ph)_3P$

(3) Cette réaction est habituellement effectuée en présence d'une base telle que le diisopropylamide de lithium, le butyllithium, l'alkoxyde de sodium ou l'hydrure de sodium.

(ii) par réarrangement et élimination de HS( O)$Z_3$ d'un composé de formule:

$$Q_2CHR_2CHR_3CR_4 = C\begin{matrix} / \ S(\rightarrow O)Z_3 \\ \backslash \ CO_2Z_4 \end{matrix}$$

dans lequel $Q_2$, $R_2$, $R_3$ et $R_4$ sont tels que définis précédemment, $Z_3$ est n'importe quel groupement approprié, par exemple phényle, phényle substitué tel que 4-chlorophényle ou alkyle en $C_{1-4}$, par exemple méthyle, $Z_4$ est alkyle en $C_{1-4}$, par exemple méthyle ou éthyle.

Le composé ci-dessus peut être obtenu par réaction d'un composé $Q_2CHR_2CHR_3CR_4O$ avec un composé $Z_3$ $S(O)CH_2CO_2Z_4$.

(iii) Par élimination d'un composé $Q_2CHR_2CR_3(OZ_5)CR_4{=}CR_{55}\text{-}CO_2Z_4$ dans lequel $Q_2$, $R_2$, $R_3$, $R_4$, $R_5$ et $Z_4$ sont tels que définis ci-dessus, et $Z_5$ est hydrogène ou acyle en $C_{1-4}$ tel que acétyle. La réaction est de préférence effectuée dans un solvant aromatique, notamment en présence d'un catalyseur de molybdène et d'une base telle que le bis-triméthylsilylacétamide.

Le composé ci-dessus peut-être obtenu par réaction d'un aldéhyde approprié avec un composé sulfényle approprié, suivie d'acylation,

(iv) réaction d'un composé de formule $Q_2CR_2{=}CR_3C({=}O)R_4$ avec un composé de formule $Me_3SiCHR_5CO_2Z_4$ dans lequel $Q_2$, $R_2$ à $R_5$, $X_1$ et $Z_4$ sont tels que définis précédemment.

Ce procédé peut être effectué dans un solvant anhydre, par exemple le tétrahydrofuranne en l'absence d'oxygène, en présence d'une base, par exemple le cyclohexylisopropylamide de lithium,

(v) par réaction d'un composé de formule $Q_2CR_2{=}CR_3\text{-}C(OZ_6){=}CR_5CO_2Z_4$ avec un composé de formule $R_4M_1$ dans lequel $Q_2$, $X_1$, $R_22$, $R_3$, $R_4$, $R_5$ et $Z_4$ sont tels que définis précédemment, $Z_6$ est un groupement approprié tel que le dialkylphosphate ou le trifluorométhanesulfonate et $M_1$ est un métal tel que le cuivre(I)

ou le cuivre(I) associé au lithium ou magnésium.

Ce procédé peut être réalisé à basse température dans un solvant éthéré anhydre tel que l'éther diéthylique, le sulfure de diméthyle ou le tétrahydrofuranne en l'absence d'oxygène,

(vi) par réaction d'un composé de formule $Q_2CR_2=CR_3M_2$ avec un composé de formule Y $CR_4=CR_5CO_2Z_4$, dans lequel $Q_2$, $X_1$, $R_2$, $R_3$, $R_4$, $R_5$ et $Z_4$ sont tels que définis précédemment, Y est halogéno ou étain et $M_2$ est un silyle ou un groupement métallifère tel que le triméthylsilyl ou un groupement contenant du zirconium, de l'étain, de l'aluminium ou du zinc, par exemple un groupement chlorure de bis(cyclopentadienyl)zirconium. Ce procédé est habituellement effectué à une température pas trop élevée, c'est-à-dire comprise entre 0° et 100°C, et notamment à la température ambiante, dans un solvant éthéré non aqueux tel que le tétrahydrofuranne, en présence d'un catalyseur de palladium(0) (tel que le bis(triphénylphosphine)palladium) et sous une atmosphère inerte d'azote ou d'argon,

(vii) par élimination de $Z_3S(\rightarrow O)H$ d'un composé de formule

$$Q_2CR_2=CR_3CHR_4\underset{\displaystyle S(\rightarrow O)Z_3}{\underset{|}{C}}R_5CO_2Z_4$$

dans lequel $Q_2$, $X_1$, $R_2$, $R_3$, $R_4$, $R_5$, $Z_3$ et $Z_4$ sont tels que définis précédemment.

Le composé ci-dessus peut être obtenu par réaction d'un composé $Q_2CHR_2CR_3=CHR_4$ avec $Z_3S(O)CH_2CO_2Z_4$.

Le procédé (b) peut être effectué par fixation d'un groupement aldéhydique ou cétonique soit à l'extrémité amide/thioamide soit au fragment $QX_1$ de formule (I) et puis en faisant réagir celui-ci avec un ylure phosphoreux approprié,

c'est-à-dire

$$Q_2(CR_2=CR_3)COR_4 + Z_2CHR_5.C(=O)NHR_1 \text{ ou}$$
$$Q_2COR_2 + Z_2CHR_3CR_4=CR_5.C(=O)NHR_1 \text{ ou}$$
$$Q_2(CR_2=CR_3)CHR_5Z_2 + R_5CO.C(=O)NHR_1$$

dans lesquels $Q_2$, $R_2$, $R_3$, $R_4$, $R_5$, $R_1$, $X_1$ et $Z_2$ sont tels que définis précédemment.

Le procédé (b) est effectué dans un solvant inerte anhydre, par exemple un éther tel que le tétrahydrofuranne, éventuellement en présence d'une base, et de préférence en l'absence d'oxygène, par exemple sous une atmosphère d'azote à basse température (-60° à 20°C). L'ylure phosphoreux peut être obtenu à partir de son précurseur tel que décrit ci-dessus par réaction avec une base tel que le diisopropylamide de lithium, le butyllithium, l'alkoxyde de sodium ou l'hydrure de sodium. Les composés de formule (I), dans laquelle X est le soufre sont de préférence préparés par le procédé (b) lorsque $Z_2$ est un groupement $(C_{1-4}\text{ alkoxy})_2P=O$. Les aldéhydes intermédiaires $Q_2CR_2=O$ peuvent être préparés par hydrolyse acide d'un cétal, un éther énolique ou un acétal dans un solvant tel que l'acétone-eau ou par oxydation des alcools appropriés à l'aide du chlorochromate de pyridinium, du dichromate de pyridinium ou du chlorure d'oxalylediméthylsulfoxide dans un solvant tel que le dichlorométhane. Les aldéhydes peuvent également être préparés par réduction des nitriles appropriés par un réactif tel que l'hydrure de diisobutylaluminium dans l'hexane.

Les alcools peuvent être préparés par

a) réaction de $Q_2CH=CX_2OH$ avec $(Z_7)_2M_2$ et $CH_2X_2^3$ et $X_2$ est un groupement tel que hydrogène, fluoro, chloro ou méthyle $X_3$ est un halogène tel que l'iode, $Z_7$ est un groupement alkyle en $C_{1-4}$ tel que éthyle et $M_2$ un métal tel que le zinc, dans un solvant inerte tel que l'hexane ou le dichlorométhane, à une température modérée (-20° à +20°) et $CH_2$ et $CH=CX_2$ s'associent pour former le noyau cyclopropane, ou

b) réduction d'un ester $Q_2CO_2Z_4$, ou de l'acide carboxylique approprié avec par exemple l'hydrure de diisobutylaluminium ou le diborane dans un solvant inerte tel que le dichlorométhane ou le tétrahydrofuranne, à température modérée (-20° à 25°). Les esters peuvent être préparés par réaction d'un diazoacétate $N_2CH.CO_2Z_4$ avec un composé $QCH=CH_2$ en présence d'un catalyseur de cuivre tel que le sulfate de cuivre où CH et $CH=CH_2$ s'associent pour former $Q_1$. Les esters peuvent également être préparés par réaction de $QCH=CHCO_2Z_4$ avec un anion dérivé de $Me_zS(O)_mC(Z_7)_2$ où $Z_7$ est hydrogène ou alkyle en $C_{1-6}$ et m est 1 ou 2.

L'alcool résultant est séparé en ses énantiomères par formation d'un mélange diastéréomérique par réaction avec un agent de résolution approprié tel que l'isocyanate de R-phénéthyle lorsque $X_1$ est fluoro, et le chlorure de camphosulfonyle lorsque $X_1$ est chloro suivi de la cristallisation fractionnée pour obtenir les diastéréoisomères désirés et du clivage de l'agent de résolution, à l'aide d'éthoxyde de sodium dans l'éthanol lorsque $X_1$ est fluoro et acétate de sodium dans le diglyme lorsque $X_1$ est chloro.

Lorsque $X_1$ est hydrogène, l'aldéhyde énantiomérique intermédiaire $QQ_1CHO$ peut encore être préparé par induction assymétrique du noyau cyclopropyle trans. Celui peut être effectué par réaction d'un composé de formule (IV), dans lequel Q est défini tel que précédemment et $Pr^i$ est isopropyle:

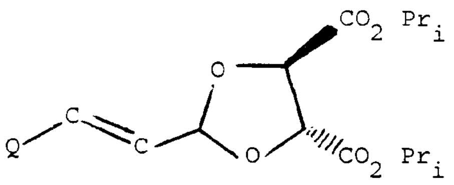

(IV)

avec le diiodométhane et le diéthylzinc dans l'hexane pour donner le noyau cyclopropane assymétrique, à savoir un composé de formule (V) dans lequel Q et $Pr^i$ sont tels que définis précédemment:

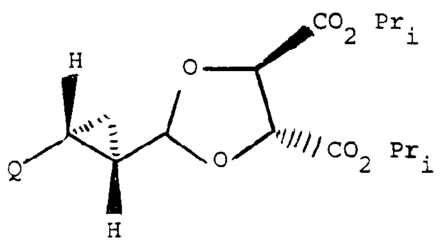

(V)

Le noyau dioxolane est alors éliminé par hydrolyse acide, par exemple à l'aide d'acide chlorhydrique dilué dans du tétrahydrofuranne. Le composé de formule (IV) peut être préparé comme présenté dans le schéma.

Les intermédiaires de la présente invention forment un autre aspect de la présente invention et peuvent éventuellement être préparés par des méthodes courantes autres que celles décrites.

Les composés de formule (I) peuvent être utilisés pour lutter entre les organismes nuisibles tels que les arthropodes, par exemple les insectes et acariens nuisibles, et les helminthes, c'est-à-dire les nématodes. Ainsi, la présente invention prévoit une méthode de lutte contre les arthropodes et/ou les helminthes comportant l'administration à l'arthropode et/ou l'helminthe ou à leur environnement, une quantité efficace pour les arthropodes d'un composé de formule (I). La présente invention a également pour objet une méthode de lutte et/ou de destruction d'infestations par les arthropodes et/ou les helminthes des animaux (dont l'homme) et/ou des plantes (dont les arbres) et/ou des produits stockés comportant l'administration à l'animal ou aux locaux, d'une quantité efficace d'un composé de formule (I). La présente invention prévoit également l'utilisation des composés de formule (I) en médecine humaine et vétérinaire, en hygiène publique et en agriculture pour la lutte contre les arthropodes et/ou les helminthes nuisibles.

Les composés de formule (I) sont particulièrement utiles pour la protection de cultures de champ, fourragères, de plantations, de serres, de vergers et de vignes, d'arbres d'ornement, de plantation et forrestiers, par exemple des céréales (telles que le maïs, le blé, le riz et le sorgho), le coton, le tabac, des légumes et des salades (telles que les haricots, le colza, les courges, la laitue, les oignons, les tomates et les poivrons), des cultures de champ (telles que la pomme de terre, la betterave sucrière, les arachides, le soja et la navette), de la canne à sucre, de l'herbage et du fourrage (telles que le maïs, le sorgho et la luzerne), des plantations (telles que le thé, le café, le cacao, la banane, le palmier, le coco, le caoutchouc et les épices), des vergers et de bosquets (telles que les fruits à noyau et à pépins, les agrumes, le kiwi, l'avocat, la mangue, les olives et les noix), des vignes, des plantes d'ornement, des fleurs et des arbustes de serre et dans les jardins et les parcs, des arbres forestiers (tant à feuilles caduques qu'à feuilles persistantes) en forêts, plantations et pépinières.

Ils sont également utiles dans la protection de bois (sur pieds, abattu, transformé, stocké ou de structure) contre l'attaque par les mouches à scie (par exemple Urocerus) ou les coléoptères (par exemple les scolytidés, les platypodidés, les lyctidés, les bostrychidés, les cérambycidés, et les anobiidés).

Ils trouvent leur application dans la protection des produits stockés tels que les céréales, les fruits, les noix, les épices et le tabac, qu'ils soient entiers, broyés ou transformés en produits, contre l'attaque par les papillons de nuit, les coléoptères et les acariens. Sont également protégés les produits animaux stockés tels que les peaux, les poils, la laine et les plumes, à l'état naturel, transformés (par exemple les moquettes ou les tissus) contre l'attaque par les papillons de nuit et les coléoptères; de même que la viande et le poisson stockés contre l'attaque par les coléoptères, les acariens et les mouches.

Les composés de formule générale (I) sont particulièrement utiles dans la lutte contre les arthropodes ou

les helminthes qui sont néfastes, ou qui transmettent ou agissent comme vecteurs de maladies chez l'homme et les animaux domestiques, par exemple ceux cités précédemment, et plus particulièrement dans la lutte contre les tiques, les acariens, les poux, les puces, les moucherons et les mouches piquantes, nuisibles et de la myiase.

Les composés de formule (I) peuvent être mis en oeuvre à de telles fins par application de manière connue des composés eux-mêmes ou à l'état dilué, sous forme de bain, de pulvérisation, de nébulisation, de laque, de mousse, de produit de poudrage, de poudre, de suspension aqueuse, de pâte, de gel, de crème, de shampooing, de graisse, de solide combustible, de natte de vaporisation, de serpentin combustible, d'appât, de complément diététique, de poudre mouillable, de granule, d'aérosol, de concentré émulsifiable, de suspension huileuse, de solution huileuse, de bombe pressurisée, de substance imprégnée, de formulation à appliquer en surface type "pour on" ou d'autres formulations standard bien connues de l'homme de l'art. Les concentrés pour bain ne sont pas appliqués tel quels mais sont dilués à l'eau et les animaux sont plongés dans un bain contenant la solution de lavage ou bain. Les pulvérisations peuvent appliquées à la main ou à l'aide d'une rampe ou un arc de pulvérisation. L'animal, le sol, la plante ou la surface sous traitement peuvent être saturés par application d'un grand volume ou recouverts susperficiellement par application de faibles ou ultra-faibles volumes. Les suspensions aqueuses peuvent être appliquées de la même manière que les pulvérisations ou les bains. Les produits de poudrage peuvent être distribués à l'aide d'une poudreuse ou, dans le cas des animaux, introduits dans des sacs perforés attachés à des arbres ou à des barres de frottement. Les pâtes, les shampooings et les graisses peuvent être appliqués manuellement à la surface d'un matériau inerte tel que celui contre lequel les animaux se frottent, transférant ainsi la matière sur leur peau. Les formulations à appliquer en surface type "pour on" sont distribuées sous forme d'une unité de liquide de faible volume sur le dos de l'animal de telle sorte que toute ou la plus grande partie du liquide reste sur l'animal.

Les composés de formule (I) peuvent être préparés soit sous forme de formulations prêtes à utiliser sur l'animal, la plante ou la surface, soit sous forme de formulations nécessitant une dilution préalable à l'application, mais les deux types de formulation comportent un composé de formule (I) en mélange intime avec un ou plusieurs supports ou diluants. Les supports peuvent être liquides, solides ou gazeux ou comporter des mélanges de telles substances, et le composé de formule (I) peut être présent à une concentration de 0,025 à 99 % p/v selon que la formulation nécessite ou non une dilution supplémentaire.

Les produits de poudrage, les poudres et les granules et autres formulations solides comportent le composé de formule (I) en mélange intime avec un support inerte solide en poudre, par exemple les argiles, le kaolin, la bentonite, l'attapulgite, le charbon noir absorbant, le talc, le mica, la craie, le gypse, le phosphate tricalcique, le liège en poudre, le silicate de magnésium, les supports végétaux, l'amidon et les terres de diatomées. De telles formulations sont généralement préparées par imprégnation des diluants solides avec des solutions du composé de formule (I) dans des solvants volatiles, par évaporation des solvants et éventuellement par broyage des produits de manière à obtenir de poudres, et éventuellement par granulation, compression ou encapsulation des produits.

Les pulvérisations du composé de formule (I) peuvent comprendre une solution dans un solvant organique (par exemple ceux énumérés ci-dessus) ou une émulsion dans l'eau (lavage en bain ou lavage par pulvérisation) préparée sur le terrain à partir d'un concentré émulsifiable (connu par ailleurs comme une huile miscible dans l'eau) qui peut également être utilisée à titre de bain. Le concentré comporte de préférence un mélange de principe actif, avec ou sans solvant organique et un ou plusieurs émulsionnants. Les solvants peuvent être présents dans de larges gammes mais de préférence dans une quantité de 0 à 90 % p/v de la composition, et peuvent être choisis parmi le kérosène, les cétones, les alcools, le xylène, le naphtalène, et d'autres solvants connus dans le domaine de la formulation. On peut faire varier la concentration des émulsionnants dans de larges gammes, mais elle est de préférence dans la fourchette de 5 à 25 % p/v et les émulsionnants sont notamment des surfactants non-ioniques dont les esters polyoxyalkyléniques d'alkylphénol et les dérivés polyoxyéthyléniques d'anhydride d'hexitol, et des surfactants anioniques dont le laurylsulfate de sodium, les sulfates d'éther d'alcool gras, les sels de sodium et de calcium de sulfonate d'alkylaryle et de sulfosuccinate d'alkyle. Les émulsionnants cationiques comprennent le chlorure de benzalkonium et les éthosulfates d'amonium quaternaire.

Les émulsionnants amphotériques comprennent l'imidazoline oléique et l'alkyldiméthylbétaïne carboxyméthylées.

Les nattes de vaporisation comportent habituellement un mélange de coton et de cellulose comprimés en plaquettes de dimension d'environ 35 × 22 × 3 mm, traitées par jusqu'à 0,3 ml de concentré comportant le principe actif dans un solvant organique et éventuellement un antioxydant, un colorant et un parfum. L'insecticide est vaporisé à l'aide d'une source de chaleur telle qu'un éléctro émanateur.

Les solides combustibles comportent habituellement de la poudre de bois et du liant mélangé avec le principe actif et formé en bandes profilées (généralement enroulées). Un colorant et un fongicide peuvent également être ajoutés. Les poudres mouillables comportent un support solide inerte, un ou plusieurs surfactants, et éventuellement des stabilisateurs et/ou antioxydants.

Les concentrés émulsifiables comportent des agents émulsionnants, et souvent un solvant organique tel que le kérosène, les cétones, les alcools, les xylènes, le naphte aromatique et d'autres solvants connus dans la technique.

Les poudres mouillables et les concentrés émulsifiables contiennent habituellement de 5 à 95 % en poids du principe actif et sont dilués, à l'eau par exemple, avant d'être utilisés.

Les laques comportent une solution de principe actif dans un solvant organique, ainsi qu'une résine et éventuellement un plastifiant.

Les solutions de lavage pour bains peuvent être préparées non seulement à partir de concentrés émulsifiables, mais également à partir de poudres mouillables, de bains à base de savon et de suspensions aqueuses comportant un composé de formule (I) en mélange intime avec un agent dispersant et un ou plusieurs surfactants.

Les suspensions aqueuses d'un composé de formule (I) peuvent comporter une suspension dans l'eau ainsi qu'un agent de suspension, un agent stabilisateur, ou d'autres agents. Les suspensions ou solutions peuvent être appliquées de manière connue, telles quelles ou à l'état dilué.

Les graisses (ou pommades) peuvent être préparées à partir d'huiles végétales, d'esters synthétiques d'acides gras, ou de suintine ainsi qu'avec une base inerte telle que de la paraffine molle. Un composé de formule (I) est de préférence uniformément distribué dans le mélange en solution ou en suspension. Les graisses peuvent également être réalisées à partir de concentrés émulsifiables en les diluant dans une pommade de base.

Les pâtes et les shampooings sont également des préparations semi-solides dans lesquelles un composé de formule (I) peut être présent sous forme d'une dispersion uniforme dans une base appropriée telle que la paraffine liquide, ou réalisée sans graisse à partir de glycérine, de mucilage ou d'un savon approprié. Comme les graisses, les shampooings et les pâtes sont habituellement appliqués sans dilution complémentaire, ils doivent contenir le pourcentage approprié du composé de formule (I) nécessaire pour le traitement.

Les sprays aérosols peuvent être préparés sous forme d'une simple solution du principe actif dans le propulseur d'aérosol et de co-solvant tel que les alkanes halogénés et les solvants mentionnés ci-dessus respectivement. Les formulations à appliquer en surface peuvent être réalisées sous forme d'une solution ou d'une suspension d'un composé de formule (I) dans un milieu liquide. Un volatile ou un mammifère peut également être protégé contre l'infestation par des ectoparasites acariens à l'aide du port d'un dispositif approprié en matière plastique moulée et profilée, imprégné d'un composé de formule (I). Comme tels dispositifs on peut citer des colliers, des plaques, des bandes, des feuilles et des bandelettes imprégnées, convenablement attachées aux endroits appropriés du corps. La matière plastique peut être un chlorure de polyvinyle (PVC).

La concentration du composé de formule (I) à appliquer sur un animal, dans des locaux ou à l'extérieur des locaux pourra varier selon le composé choisi, l'intervalle entre les traitements, la nature de la formulation et l'infestation possible, mais en général 0,001 à 20.0 % p/v et de préférence 0,01 à 10 % du composé doit être présent dans la formulation appliquée. La quantité de composé déposée sur un animal varie selon la méthode d'application, la taille de l'animal, la concentration du composé dans la formulation appliquée, le facteur de dilution de la formulation et la nature de la formulation, mais elle sera en général comprise dans la gamme de 0,0001 % à 0,5 % p/p, à l'exception de formulations non diluées telles que les formulations à appliquer en surface qui seront en général déposées à une concentration dans la gamme de 0,1 à 20,0 % et de préférence 0,1 à 10 %. La quantité de composé à appliquer aux produits stockés sera en général comprise dans la gamme de 0,1 à 20 ppm. Les sprays pour l'espace peuvent être appliqués pour donner une concentration initiale moyenne de 0,001 à 1 mg de composé de formule (I) par mètre cube d'espace traité.

Les composés de formule (I) sont également mis en oeuvre pour la protection et le traitement d'espèces végétales, dans quel cas, une quantité efficace, du point de vue insecticide, acaricide ou nématicide, du principe actif est appliquée. Le taux d'application pourra varier selon le composé choisi, la nature de la formulation, le mode d'application, l'espèce végétale, la densité de peuplement et l'infestation possible, et d'autres facteurs similaires, mais en général, un taux d'utilisation appropriée pour les cultures agricoles est compris dans la gamme de 0,001 à 3 kg/Ha et de préférence entre 0,01 et 1 kg/Ha. Des formulations types à utilisation agricole contiennent entre 0,0001 % et 50 % d'un composé de formule (I) et notamment entre 0,1 et 15 % en poids d'un composé de formule (I).

Les formulations de produits de poudrage, de graisses, de pâtes, et d'aérosols sont habituellement appliquées de manière aléatoire comme décrites ci-dessus et on peut mettre en oeuvre des concentrations de 0,001 à 20 % p/v d'un composé de formule (I) dans la formulation appliquée.

Il a été trouvé que les composés de formule (I) ont une activité contre la mouche domestique commune (Musca domestica). De plus, certains composés de formule (I) ont une activité contre d'autres arthropodes

nuisibles dont Myzus persicae, Tetranychus urticae, Plutella xylostella, Culex spp. Tribolium castaneum, Sitophilus granarius, Periplaneta ameircana et Blattella germanica. Les composés de formule (I) sont ainsi utiles dans la lutte contre les arthropodes, par exemple les insectes et les acariens, dans n'importe quel environnement où ceux-ci constituent des organismes nuisibles, par exemple en agriculture, en élevage d'animaux, pour contrôler l'hygiène publique et en situation domestique.

Les insectes nuisibles comprennent des membres des ordres coléoptères (par exemple Anobium, Ceutorhynchus, Rhynchophorus, Cosmopolites, Lissorhoptrus, Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus ou Anthrenus spp.), Lepidoptères (par exemple Ephestia, Mamestra, Earias, Pectinophora, Ostrinia, Trichoplusia, Pieris, Laphygma, Agrotis, Amathes, Wiseana, Tryporysa, Diatraea, Sporganothis, Cydia, Archips, Plutella, Chilo, Heliothis, Spodoptera ou Tineola spp.), Diptères (par exemple, Musca, Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma, Hylemyia, Atherigona, Chlorops, Phytomyza, Ceratitis, Liriomyza et Melophagus spp.), Phthiraptères (Malophaga par exemple, Damalina spp. et Anoplura par exemple Linognathus et Haematopinus spp.), Hemiptères (par exemple Aphis, Bemisia, Phorodon, Aeneolamia, Empoasca, Parkinsiella, Pyrilla, Aonidiella, Coccus, Pseudococus, Helopeltis, Lygus, Dysdercus, Oxycarenus, Nezara, Aleurodes, Triatoma, Psylla, Mysus, Megoura, Phylloxera, Adelyes, Niloparvata, Nephrotetix ou Cimex spp.), Orthoptères (par exemple Locusta, Gryllus, Schistocerca ou Acheta spp.), Dictyoptères (par exemple Blattella, Periplaneta ou Blatta spp.), Hyménoptères (par exemple Athalia Cephus Atta, Solenopsis ou Monomorium spp.), Isoptères (par exemple Odontotermes et Reticulitermes spp.), Siphonaptères (par exemple Ctenocephalides ou Pulex spp.), Thysanura (par exemple Lepisma spp.), Dermaptères (par exemple Forficula spp.), Pscoptères (par exemple Peripsocus spp.) et Thysanoptères (par exemple Thrips tabaci).

Les acariens nuisibles comprennent les tiques, par exemple des membres des genres Boophilus, Ornithodorus, Rhipicephalus, Amblyomma, Hyalomma, Ixodes, Haemaphysalis, Dermacentor et Anocentor, et les acariens et gales tels que Acarus, Tetranychus, Psoroptes, Notoednes, Sarcoptes, Psorergates, Chorioptes, Eutrombicula, Demodex, Panonychus, Bryobia, Eriophyes, Blaniulus, Polyphagotarsonemus, Scutigerella, et Oniscus spp.

Les nématodes qui attaquent les plantes et les arbres importants dans le domaine agronomique, forestier et horticole, soit directement ou par propagation de maladies bactériennes, virales, à mycoplasmes ou fongiques des plantes, comprennent les nématodes cécidogènes tels que Meloidogyne spp. (par exemple M. incognita); les nématodes kystiques tels que Globodera spp. (par exemple G. rostochiensis); Heterodera spp. (par exemple hydrogen. avenae); Radopholus spp. (par exemple R. similis); les nématodes de lésion tels que Pratylenchus spp. (par exemple P. pratensis); Belonolaimus spp. (par exemple B. gracilis); Tylenchulus spp. (par exemple T. semipenetrans); Rotylenchulus spp. (par exemple R. reniformis); Rotylenchus spp. (par exemple R. robustus); Helicotylenchus spp. (par exemple hydrogen multicinctus); Hemicycliophora spp. (par exemple hydrogen gracilis); Criconemoides spp. (par exemple C. similis); Trichodorus spp. (par exemple T. Primitivus); les nématodes stylets tels que Xiphinema spp. (par exemple X. diversicaudatum), Longidorus spp. (par exemple L. elongatus); Hoplolaimus spp. (par exemple hydrogen. coronatus); Aphelenchoides spp. (par exemple A. ritzema-bosi, A. besseyi); les nématodes des tiges et des bulbes tels que Ditylenchus spp. (par exemple D. dipsaci).

Les composés de l'invention peuvent être associés à un ou plusieurs autres principes actifs du point de vue pesticide (par exemple des pyréthrinoïdes, des carbamates et des organophosphates) et/ou à des attractifs, des répulsifs, des bactéricides, des fongicides, des nématicides, des anthélmintiques, etc. De plus, il a été trouvé que l'activité des composés de l'invention peut être améliorée par l'addition d'un synergiste ou d'un potentialisateur, par exemple: un des synergistes de la classe d'inhibiteurs d'oxydase tel que le butoxyde de pipéronyle ou le 2-propynylphénylphosphonate de propyle; un deuxième composé de l'invention; ou un composé pesticide pyréthrinoïde. Lorsqu'un synergiste inhibiteur d'oxydase est présent dans une formule de l'invention, le rapport du synergiste au composé de formule (I) sera dans la gamme 25:1-1:25, par exemple d'environ 10:1.

Les stabilisateurs pour prévenir toute dégradation chimique pouvant se produire avec les composés de l'invention comprennent par exemple des antioxydants (tels que les tocophéroles, le butylhydroxyanisole et le butylhydroxytoluène) et des adsorbants (tels que l'épichlorhydrine) et des bases organiques et minérales par exemple les trialkylamines, tels que la triéthylamine, qui peuvent agir comme stabilisateurs basiques et comme pièges.

Applicabilité industrielle

Les composés de la présente invention présentent une activité pesticide.
Les exemples suivants illustrent de manière non-limitative les aspects préférés de l'invention.

Formulations

1. Concentré émulsifiable

| | |
|---|---|
| Composé de formule (I) | 10,00 |
| Ethoxylate d'alkylphénol* | 7,50 |
| Sulfonate d'alkylaryle* | 2,50 |
| Solvant aromatique en $C_{8-13}$ | 80,00 |
| | 100,00 |

2. Concentré émulsifiable

| | |
|---|---|
| Composé de formule (I) | 10,00 |
| Ethoxylate d'alkylphénol* | 2,50 |
| Sulfonate d'alkylaryle* | 2,50 |
| Solvant cétonique | 64,00 |
| Solvant aromatique en $C_{8-13}$ | 18,00 |
| Antioxydant | 3,00 |
| | 100,00 |

3. Poudre mouillable

| | |
|---|---|
| Composé de formule (I) | 5,00 |
| Solvant aromatique en $C_{8-13}$ | 7,00 |
| Solvant aromatique en $C_{18}$ | 28,00 |
| Argile blanche | 10,00 |
| Sulfonate d'alkylaryle* | 1,00 |
| Acide naphtalènesulfonique | 3,00 |
| Terre de diatomée | 46,00 |
| | 100,00 |

4. Produit de poudrage

| | |
|---|---|
| Composé de formule (I) | 0,50 |
| Talc | 99,50 |
| | 100,00 |

5. Appât

| | |
|---|---|
| Composé de formule (I) | 0,5 |
| Sucre | 79,5 |
| Cire de paraffine | 20,0 |
| | 100,00 |

6. Concentré en émulsion

| | |
|---|---|
| Composé de formule (I) | 5,00 |
| Solvant aromatique en $C_{8-13}$ | 32,00 |
| Alcool cétylique | 3,00 |
| Monooléate de glycérol polyoxyéthylénique* | 0,75 |
| Esters de sorbitan polyoxyéthyléniques* | 0,25 |
| Solution de silicone | 0,1 |
| Eau | 58,9 |
| | 100,00 |

7. Concentré en suspension

| | |
|---|---|
| Composé de formule (I) | 10,00 |
| Ethoxylate d'alkylaryle* | 3,00 |
| Solution de silicone | 0,1 |
| Alkanediol | 5,0 |
| Silice de combustion | 0,50 |
| Gomme de xanthane | 0,20 |
| Eau | 80,0 |
| Tampon | 1,2 |
| | 100,00 |

8. Microémulsion

| | |
|---|---|
| Composé de formule (I) | 10,00 |
| Monooléate de glycérol polyoxyéthyléné* | 10,00 |
| Alkanediol | 4,00 |
| Eau | 76,00 |
| | 100,00 |

9. Granules dispersibles dans l'eau

| | |
|---|---|
| Composé de formule (I) | 70,00 |
| Polyvinyle pyrrolidine | 2,50 |
| Ethoxylate d'alkylaryle | 1,25 |
| Sulfonate d'alkylaryle | 1,25 |
| Argile blanche | 25,00 |
| | 100,00 |

10. Granules

| | |
|---|---|
| Composé de formule (I) | 2,00 |
| Ethoxylate d'alkylphénol* | 5,00 |
| Sulfonate d'alkylaryle* | 3,00 |
| Solvant aromatique en $C_{8-13}$ | 20,00 |
| Granules de kieselguhr | 70,00 |
| | 100,00 |

11. Aerosol (bombe pressurisée)

| | |
|---|---|
| Composé de formule (I) | 0,3 |
| Butoxide de pipéronyle | 1,5 |
| Solvant d'hydrocarbure saturé en $C_{8-13}$ | 58,2 |
| Butane | 40,0 |
| | 100,00 |

12. Aérosol (bombe pressurisée)

| | |
|---|---|
| Composé de formule (I) | 0,3 |
| Solvant d'hydrocarbure saturé en $C_{8-13}$ | 10,0 |
| Monooléate de sorbitan | 1,0 |
| Eau | 40,0 |
| Butane | 48,7 |
| | 100,00 |

13. Aérosol (bombe pressurisée)

| | |
|---|---|
| Composé de formule (I) | 1,00 |
| $CO_2$ | 3,00 |
| Monooléate de glycérol polyoxyéthyléné* | 1,40 |
| Propanone | 38,00 |
| Eau | 56,60 |
| | 100,00 |

14. Laque

| | |
|---|---|
| Composé de formule (I) | 2,50 |
| Résine | 5,00 |
| Antioxydant | 0,50 |
| Essence blanche fortement aromatique | 92,00 |
| | 100,00 |

15. Bouillie (prête à l'emploi)

| | |
|---|---|
| Composé de formule (I) | 0,10 |
| Antioxydant | 0,10 |
| Kérosène inodore | 99,8 |
| | 100,00 |

16. Bouillie potentiallisée (prête à l'emploi)

| | |
|---|---|
| Composé de formule (I) | 0,10 |
| Butoxyde de pipéronyle | 0,50 |
| Antioxydant | 0,10 |
| Kérosène inodore | 99,30 |
| | 100,00 |

17. Microencapsulées

| | |
|---|---|
| Composé de formule (I) | 10,0 |
| Solvant aromatique en $C_{8-13}$ | 10,0 |
| Di-isocyanate aromatique# | 4,5 |
| Ethoxylate d'alkylphénol* | 6,0 |
| Diamine d'alkyle* | 1,0 |
| Diéthylènetriamine | 1,0 |
| Acide chlorhydrique concentré | 2,2 |
| Gomme de xanthane | 0,2 |
| Silice de combustion | 0,5 |
| Eau | 64,6 |
| | 100,00 |

*= Surfactant

#= Réagit pour former les parois polyuréiques de la microcapsule

L'antioxydant peut être n'importe lequel des composés suivants, pris séparément ou ensemble,
Butylhydroxytoluène
Butylhydroxyanisole
Vitamice C (acide ascorbique)

Les exemples suivants illustrent de manière non limitative les aspects préférés de l'invention.

PROTOCOLE EXPERIMENTAL

Méthodes de synthèse et procédés généraux:

Divers composés ont été synthétisés et caractérisés en conformité avec les protocoles expérimentaux suivants.

Les spectres de RMN de $^{1}H$. ont été obtenus à l'aide d'un spectromètre Burker AM-250 dans des solutions de deutérochloroforme en utilisant le tétraméthylsilane comme étalon interne et sont exprimés en ppm à partir du TMS, nombre de protons, nombre de pics, constante de couplage J Hz.

L'évolution des réactions peut être commodément suivie sur des feuilles d'aluminium (40 × 80 mm) préalablement recouvertes de couches de gel de silice de 0,25 mm avec un révélateur fluorescent et révélées dans un solvant ou un mélange de solvants appropriés. Les températures sont toujours en degrés Celsius.

Les opérations classiques ont été réalisées de la manière suivante:

Le mélange réactionnel a été partagé entre un solvant organique et de l'eau. Les phases ont été séparées et la phase organique lavée avec au moins un volume équivalent de base aqueuse diluée de manière appropriée, et puis avec une solution saline saturée. La phase organique a alors été séchée dans un dessiccateur, convenablement sur le sulfate de magnésium, et filtrée. Les solvants volatiles ont été éliminés et le produit résultant soumis à une purification appropriée et utilisée dans l'étape suivante de la synthèse ou analysé en tant que produit final.

L'aldhéhyde, l'acide cinnamique et l'amine de départ ont été obtenus d'Aldrich, de BDH, de Fluorochem, Fluka ou de Lancaster Synthesis à l'exception des composés suivants dont la préparation est décrite ci-dessous.

Exemple 1

(-)-(2E,4E)-N-(2-Méthylprop-2-ényl)-5-[(1R,2S)-c-2-(3,4-dibromophényl)-r-1-fluorocyclopropyl] 3-méthylpenta-2,4-diénamide (Composé 1)

Le (+/-)-c-2-(3,4-Dibromophényl)-r-1-fluorocyclopropylméthanol (11 g) et le (+)-(R)-α-méthylbenzylisocyanate (5 g) ont été agités à 80°C pendant 60 heures sous azote. Le produit brut résultant a été soumis à une chromatographie sur silice suivie de cristallisation fractionnée pour donner le [(1R,2R)-c-2-(3,4-dibromophényl)-r-1-fluoro-cylopropylméthyl]-(αR)-α-méthylbenzyl-carbamate (2,0 g, F 94,3°C) et le [(1S,2S)-c-1-fluoro-r-2-(3,4-dibromophényl)cyclopropylméthyl]-(αR)-α-méthylbenzyl-carbamate (3,0 g, F 118,6°C) en plus de 6,25 g du mélange. Les stéréochimies absolues ont été confirmées par rayons X.

Le [(1S,2S)-c-2-(3,4-dibromophényl)-r-1-fluorocyclopropylméthyl]-(αR)-α-méthylbenzyl-carbamate (3,7 g) a été ajouté à une solution d'éthylate de sodium (vendue par Aldrich) (0,11 g) dans de l'éthanol (2 ml) sous azote et le mélange a été porté à 70°C pendant 30 minutes. Le mélange a été refroidi et concentré sous vide. La chromatographie sur silice a donné le (-)-(1S,2S)-c-2-(3,4-dibromophényl)-r-1-fluorocyclopropylméthanol 2,42 g, F 82°C, [$\alpha_D$] -28,9°, c. 1,00 (EtOH)).

Le chlorure d'oxalyle (vendu par Aldrich) (0,72 ml) a été dissout dans du dichlorométhane (10 ml) et refroidi jusqu'à -70°C sous azote. Le diméthylsulfoxide (1,17 ml) (vendu par BDH) dans du dichlorométhane (1 ml) a été ajouté goutte à goutte. Après 5 minutes, l'alcool ci-dessus (2,42 g) dans du dichlorométhane (5 ml) a été ajouté et la suspension agitée à -70°C pendant trente minutes. La triéthylamine (vendue par Aldrich) (5,2 ml) a été ajoutée et on a laissé le mélange s'équilibrer à 0°C sur une heure. Les opérations classiques ont donné le (-)-(1S,2S)-c-2-(3,4-dibromophényl)-r-1-fluorocyclopropylméthanal (2,4 g, [$\alpha_D$] -69,7° (c 1,00 EtOH)).

Une solution de diisopropylamide de lithium dans du tétrahydrofuranne anhydre, préparée à partir du n-butyllithium (vendu par Aldrich) (4,7 ml) et de la diisopropylamine (vendue par Aldrich) (1,1 ml), a été traitée à -60°C avec du 3-méthylphosphonocrotonate de triéthyle (1,97 g) dans du tétrahydrofuranne sous azote. Après 2 heures à -60°C, l'aldéhyde ci-dessus (2,4 g) a été ajouté. Après 18 heures à 25°C, le mélange a été partagé entre de l'éther et de l'eau et la fraction éthérée traitée comme ci-dessus. La purification par chromatographie sur silice (éther/hexane) a donné le (-)-(2E/Z,4E)-éthyl-5-[(1R,2S)-c-2-(3,4-dibromophényl)-r-1-fluorocyclopropyl]-3-méthylpenta-2,4-diénoate, (2,82 g, 2E:2Z, 3:2. [$\alpha_D$] -136° (c. 1,00, EtOH)).

A l'ester ci-dessus (2,76 g) dans de l'éthanol (100 ml) a été ajouté à une solution d'hydroxide de potassium (vendue par BDH) (1,25 g) dans de l'eau (25 ml). Après 18 heures à température ambiante, la solution a été concentrée sous vide. Le résidu du sel d'acide carboxylique a été dissout dans une faible quantité d'eau et acidifiée avec de l'acide chlorhydrique concentré. L'extraction à l'éther, le séchage sur sulfate de magnésium

et la concentration sous vide ont donné l'acide (-)-(2E/Z,4E)-5-[(1S,2R)-c-2-(3,4-dibromophényl)-r-1-fluorocyclopropyl]-3-méthylpenta-2,4-diénoïque (2,45 g, $[\alpha]_D$ -2,45 g (c. 1,00, EtOH)).

A une suspension agitée de l'acide ci-dessus (2,4 g) dans du dichlorométhane (60 ml) sous azote à 0°C a été ajouté du chlorure d'oxalyle (0,63 ml) suivi d'une goutte de diméthylformamide. Après 2 heures à température ambiante, le solvant a été éliminé sous vide. Le résidu a été mis en suspension dans de l'éther (60 ml) et refroidi jusqu'à 0°C, la triéthylamine (1,0 ml) et la 2-méthylprop-2-énylamine (0,65 ml, vendue par Aldrich) ont été ajoutées et le mélange agité pendant 18 heures à température ambiante. De l'eau a été ajoutée et le mélange traité de manière classique. La chromatographie sur silice (éther/hexane) a donné le composé du titre (0,6 g, F 125°C, $[\alpha]_D$ -171° (c. 1,00, EtOH)).

Le (-)-(2E/Z,4E)-N-(2-méthylprop-2-ényl)-5-[(1R,2S)-c-2-(3,4) dibromophényl)-r-1-fluorocyclopropyl]-3-méthylpenta-2,4-diénamide, a également été isolé (Composé 2) (1,4 g, 2E:4E 3:2. $[\alpha]_D$ -132° (c. 1,00, EtOH)).

En utilisant les mêmes procédés expérimentaux, le [(1R,2R)-c-2-(3,4-dibromophényl)-r-1-fluorocyclopropylméthyl]-(αR)-a-méthylbenzyl-carbamate a été transformé en (+)-(2E,4E)-N-(2-méthylprop-2-ényl)-5-((1S,2R)-c-(3,4-dibromophényl)-r-1-fluorocyclopropyl]-3-méthylpenta-2,4-diénamide, (Composé 3) (0,11 g, $[\alpha_D]$ +170° (c. 1,00 EtOH)) et (+)-(2E/Z,4E)-N-(2-méthylprop-2-ényl)-5-[(1S,2R)-c-2-(3,4-dibromophényl)-r-fluorocyclopropyl]-3-méthylpenta-2,4-diénamide, (Composé 4) (0,78 g, 2E:2Z 5:4, $[\alpha_D]$ +131° (c. 1,00 EtOH)).

Example 2

Le (+)-(2E,4E)-N-isobutyl-5-[(1S,2R)-c-2-(3,4-dichlorophényl)-r-1-chlorocyclopropyl]-3-méthylpenta-2,4-diénamide, (Composé 5).

Le (+/-)-c-2-(3,4-dichlorophényl)-r-1-chlorocyclopropylméthanol (8,0 g) dans du dichlorométhane (160 ml) sous azote à - 20°C a été traité successivement avec la triéthylamine (5,3 ml) et le chlorure de (-)-(1S)-10-Camphosulfonyle (8,78 g, vendu par Aldrich). Après 18 heures à -5°C, le mélange a été versé dans de l'eau et traité de manière classique pour donner le sulfonate de [(1S,2S)-(-)-c-2-(3,4-dichlorophényl)-r-1-chlorocyclopropylméthyl]-(1S)-10-camphre (4,8 g, $[\alpha_D]$ -11,3° (c. 1,00, $CHCl_3$)) et le sulfonate de [(1R,2R)-(+)-c-2-(3,4-di-chlorophényl)-r-1-chlorocyclopropylméthyl]-(1S)-10-camphre (4,7 g, $[\alpha_D]$ +11,3° (c. 1,00, $CHCl_3$)). 2,7 g du mélange diastéréoisomère ont également été isolés. Les stéréochimies absolues ont été déterminées par radiocristallographie.

A une suspension agitée de sulfonate de (+)-[(1R,2R)-c-2-(3,4-dichlorophényl)-r-1-chlorocyclopropylméthyl]-(1S)-10-camphre (2,66 g) dans du 2-méthoxyéthanol (5 ml) (vendu par Aldrich) a été ajouté de l'acétate de sodium (2 g) (vendu par Aldrich) et le mélange porté à reflux pendant 7 heures. Le mélange a été refroidi, dilué à l'eau et extrait à l'éther. L'opération classique et la chromatographie (silice, éther/hexane) ont donné le (+)-(1R,2R)-c-2-(3,4-dichlorophényl)-r-1-cyclopropylméthanol sous forme d'huile incolore (0,5 g, $[\alpha_D]$ +28°, c. 1,00 (EtOH)).

Le chlorure d'oxalyle (0,19 ml) a été dissout dans du dichlorométhane (3 ml) et refroidi jusqu'à -70°C sous azote. Le diméthylsulfoxyde (0,31 ml) dans du dichlorométhane (1 ml) a été ajouté goutte à goutte. Après 5 minutes, l'alcool ci-dessus (0,5 g) dans du dichlorométhane (1 ml) a été ajouté et la suspension agitée à -70°C pendant trente minutes. La triéthylamine (1,38 ml) a été ajoutée et on a laissé le mélange s'équilibrer à 0°C sur une heure. L'opération classique a donné le (+)-(1R,2RS)-c-2-(3,4-dichlorophényl)-r-1-chlorocyclopropylméthanal (0,49 g).

Une solution de diisopropylamide de lithium dans du tétrahydrofuranne anhydre, préparée à partir du n-butyllithium (1,24 ml) et de la diisopropylamine (0,28 ml), a été traitée à -60°C avec le 3-méthylphosphonocrotonate de triéthyle (0,52 g) dans du tétrahydrofuranne sous azote. Après 2 heures à -60°C, l'aldhéhyde ci-dessus (0,49 g) a été ajouté. Après 18 heures à 25°C, le mélange a été partagé entre de l'éther et de l'eau et la fraction éthérée traitée comme ci-dessus. La purification par chromatographie sur silice (éther/hexane) a donné le (+)-(2E/Z,4E)-éthyl-5-[(1S,2R)-c-2-(3,4-dichlorophényl)-r-1-chlorocyclopropyl]-3-méthylpenta-2,4-diénoate, 90,7 g, 2E:2Z, 7:3).

A l'ester ci-dessus (0,7 g) dans de l'éthanol (10 ml) a été ajoutée une solution d'hydroxyde de potassium (0,38 g) dans de l'eau (2,5 ml). Après 18 heures à température ambiante, la solution a été concentrée sous vide. Le résidu du sel d'acide carboxylique a été dissout dans une faible quantité d'eau et acidifié avec l'acide chlorhydrique concentré. L'extraction à l'éther, le séchage sur le sulfate de magnésium et la concentration sous vide ont donné l'acide (+)-(2E/Z,4E)-5-[(1R,2S)-c-2-(3,4-dichlorophényl)-r-1-chlorocyclopropyl]-3-méthylpenta-2,4-diénoïque.

A l'acide ci-dessus (0,58 g) dans du dichlorométhane (5 ml) à 25°C sous azote, ont été ajoutés successivement de la triéthylamine (0,49 ml), du N-phénylphosphoramidochloridate (vendu par Aldrich) (0,47 g) et de l'isobutylamine (vendu par Aldrich, 0,17 ml). Après 18 heures à température ambiante, le mélange a été soumis

à une opération classique pour donner, après chromatographie (silice, éther/hexane), le composé du titre (0,23 g, F 114°C, [$\alpha_D$] +164° (c. 1,00 EtOH).

Par un procédé identique à celui décrit ci-dessus, le (-)-(2E,4E)-N-isobutyl-5-((1R,2S)-c-2-(3,4-dichlorophényl)-r-1-chlorocyclopropyl]-3-méthylpenta-2,4-diénamide, (Composé 6) a été préparé à partir du sulfonate de (-)-[(1S,2S)-c-2-(3,4-dichlorophényl)-r-1-chlorocyclopropylméthyl]-(1S)-10-camphre (0,3 g, F 114°C, [$\alpha_D$] -164° (c. 1,00, EtOH).

En utilisant les mêmes procédés expérimentaux, les composés suivants ont été préparés à partir de l'acide (-)-(2E/Z,4E)-5-[(1R,2S)-c-2-(3,4-dichlorophényl)-r-1-chlorocyclopropyl]-3-méthylpenta-2,4-diénoïque en utilisant la (S)-(+)-sec-butylamine et la (R)-(-)-sec-butylamine (vendues par Aldrich) à la place de l'isobutylamine.

(-)-(2E,4E)-N-(S-1-Méthylpropyl)-5-[(1R,2S)-c-2-(3,4-dichlorophényl)-r-1-chlorocyclopropyl]-3-méthylpenta-2,4-diénamide, (Composé 7).

(0,146 g, [$\alpha_D$] -141° (c. 1,00, EtOH)).

(-)-(2E,4E)-N-(R-1-Méthylpropyl)-5-[(1R,2S)-c-2-(3,4-dichlorophényl-r-1-chlorocyclopropyl]-3-méthylpenta-2,4-diénamide, (Composé 8).

(0,119 g [$\alpha_D$] - 186° (c. 1,00 EtOH)).

Exemple 3

(-)-(2E,4E)-N-Isobutyl-5-[(1R,2S)-trans-2-(3,4-dibromophényl)cyclopropyl]-3-méthylpenta-2,4-diénamide, (Composé 9).

Le composé du titre a été préparé par un procédé analogue à celui mis en oeuvre dans EP 369 762, exemple 12, composé 56. Le 3,4-dibromobenzaldéhyde et le 3-méthylphosphonocrotonate de triéthyle ont été utilisés à la place du 3,4-dibromobenzaldéhyde et du phosphonocrotonate de triéthyle (0,324 g, [$\alpha_D$] - 188° (c. 0,99, EtOH)).

Le (+)-(2E,4E)-N-isobutyl-5-((1R,2S)-trans-2-(3,4-dibromophényl)cyclopropyl]-3-méthylpenta-2,4-diénamide, (Composé 10), a été préparé de manière analogue en utilisant le tartrate de D-(-)-diisopropyle à la place du tartrate de L-(+)-diisopropyle (0,23 g, [$\alpha_D$] +154° (c. 0,94, EtOH)).

Les données de RNM

Composé

1. 7.58(2H,m), 7.04(1H,dd), 6.38(1H,d), 5.84(1H,dd), 5.73(1H,s), 5.59(1H,m), 4.82(2H,s), 3.90(2H,d), 2.31(3H,d), 2.29(1H,m), 1.78(1H,m), 1.76(3H,s), 1.55(1H,m).
2. 7.85 & 6.39(1H,d), 7.58(2H,m), 7.08(1H,dd), 6.11 & 5.83(1H,dd), 5.77 & 5.68(1H,d), 5.60(1H,m), 4.88(2H,s), 3.89(2H,m), 2.30 & 2.00(3H,d), 2.25(1H,m), 1.70(1H,m), 1.76(3H,s), 1.55(1H,m).
3. 7.58(2H,m), 7.05(1H,dd), 6.39(1H,d), 5.86(1H,dd), 5.73(1H,s), 5.58(1H,m), 4.85(2H,s), 3.90(2H,d), 2.31(3H,d), 2.28(1H,m), 1.78(1H,m), 1.76(3H,s), 1.55(1H,m).
4. 7.85 & 6.39(1H,d), 7.58(2H,m), 7.08(1H,dd), 6.11 & 5.83(1H,dd), 5.77 & 5.68(1H,d), 5.60(1H,m), 4.88(2H,s), 3.89(2H,m), 2.30 & 2.00(3H,d), 2.25(1H,m), 1.70(1H,m), 1.76(3H,s), 1.55(1H,m).
5. 7.40(1H,d), 7.32(1H,d), 7.09(1H,dd), 6.43(1H,d), 5.92(1H,d), 5.77(1H,s), 5.55(1H,m), 3.14(2H,t), 2.48(1H,dd), 2.30(3H,d), 1.77(3H,m), 0.93(6H,d).
6. 7.40(1H,d), 7.32(1H,d), 7.09(1H,dd), 6.43(1H,d), 5.92(1H,d), 5.77(1H,s), 5.55(1H,m), 3.14(2H,t), 2.48(1H,dd), 2.30(3H,d), 1.77(3H,m), 0.93(6H,d).
7. 7.40(1H,d), 7.31(1H,d), 7.08(1H,dd), 6.41(1H,d), 5.92(1H,d), 5.70(1H,s), 5.26(1H,$d_{large}$), 4.00(1H,m), 2.47(1H,dd), 2.29(3H,d), 1.72(2H,m), 1.42(2H,m) 1.13(3H,d), 0.92(3H,t).
8. 7.40(1H,d), 7.31(1H,d), 7.08(1H,dd), 6.41(1H,d), 5.92(1H,d), 5.70(1H,s), 5.26(1H,$d_{large}$), 4.00(1H,m), 2.47(1H,dd), 2.29(3H,d), 1.72(2H,m), 1.42(2H,m) 1.13(3H,d), 0.92(3H,t).
9. 7.49(1H,d), 7.33(1H,d), 6.88(1H,dd), 6.15(1H,d), 5.71(1H,m), 5.63(1H,dd), 5.61(1H,s), 3.12(2H,t), 2.23(3H,s), 1.95(1H,m), 1.75(2H,m), 1.26(2H,m). 0.91(6H,d).
10. 7.49(1H,d), 7.33(1H,d), 6.88(1H,dd), 6.15(1H,d), 5.71(1H,m), 5.63(1H,dd), 5.61(1H,s), 3.12(2H,t), 2.23(3H,s), 1.95(1H,m), 1.75(2H,m), 1.26(2H,m). 0.91(6H,d).

DONNEES BIOLOGIQUES

Les exemples suivants illustrent de manière non-limitative l'activité pesticide des composés de formule (I):

EXEMPLE A - ESSAIS DE PULVERISATION

L'activité des composés de l'invention a été testée par dissolution des composés dans de l'acétone (5 %) suivie de dissolution de l'eau: "Synperonic" (94,5 %:0,5 %) pour donner une émulsion d'eau. La solution a ensuite été utilisée pour traiter les insectes suivants, pour lesquels l'activité a été observée pour les fréquences de pulvérisation suivantes:

Spodoptera littoralis

Des feuilles non infectées ont été pulvérisées avec la solution à tester contenant le composé, et on a laissé sécher. Elles ont alors été infestées par 10 larves récemment écloses. La mortalié a été déterminée après 3 jours.

Les composés suivants ont été actifs à 250 ppm ou inférieur:
3, 4, 6, 5, 9, 10, 7 et 8.

Les composés suivants ont été actifs à 50 ppm ou inférieur:
1, 2.

EXEMPLE B - ESSAIS D'APPLICATION TOPIQUE

Blattella germanica

0,5 µl d'une solution du composé dans du butanone (avec ou sans butoxyde de pipéronyle) a été administré par voie topique à B. germanica mâle. La mortalité a été déterminée au bout de 6 jours.

Les composés suivants ont été actifs à 10 µg ou inférieur (+ 10 µg de butoxyde de pipéronyle):
6, 7, 8, 9 et 10.

Administration par voie topique à la mouche domestique (Musca domestica)

Les composés ont été administrés par voie topique à Musca domestica dans une solution de butanone, soit seuls soit en association avec un synergiste (6 µg de butoxyde de pipéronyle). Les mouches ont été maintenues dans de l'eau sucrée et la mortalité a été déterminée au bout de 48 heures.

Les composés suivants ont été actifs à 1 µg ou inférieur (+ 6 µg de butoxyde de pipéronyle)
3, 4, 7, et 8.

Les composés suivants ont été actifs à 0,2 µg ou inférieur (+ 6 µg de butoxyde de pipéronyle):
1, 2, 6, 9 et 10.

## Revendications

**1)** Composé de formule (II):

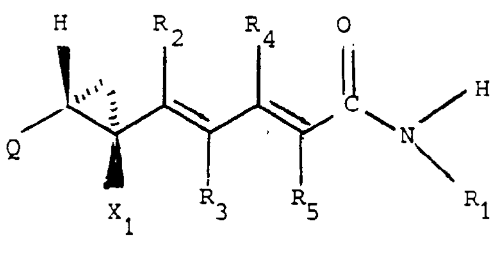

(I)

ou un sel de celui-ci, dans lequel Q est un noyau aromatique monocyclique ou un système de noyau bicyclique fusionné dont au moins un cycle est aromatique contenant 9 ou 10 atomes dont l'un des atomes peut être un azote et les autres des atomes de carbone, chaque cycle étant éventuellement substitué, ou Q est un groupement dihalogénovinyle, ou un groupement $R_6$-C≡C- dans lequel $R_6$ est un radical alkyle en $C_{1-4}$, tri(alkyle en $C_{1-4}$)silyle ou un atome d'halogène ou d'hydrogène; $R_2$, $R_3$, $R_4$ et $R_6$ sont identiques ou différents, l'un au moins étant un atome d'hydrogène et les autres étant choisis indépendamment parmi les atomes d'hydrogène, d'halogéne, les radicaux alkyle en $C_{1-4}$ ou halogénoalkyle en $C_{1-4}$; et $R_1$ est un atome d'hydrogène ou un radical

hydrocarboné en $C_{1-8}$ éventuellement substitué par dioxalanyle, halogéno, cyano, trifluorométhyle, trifluorométhylthio ou alkoxy en $C_{1-6}$ et $X_1$ est un atome d'hydrogène, de fluor ou de chlore.

**2)** Composé de formule (I) selon la revendication 1, dans lequel Q est un groupement phényle, pyridyle, thiényle ou naphtyle ou un groupement phényle, pyridyle, thiényle ou naphtyle substitué.

**3)** Composé de formule (I) selon la revendication 1, dans lequel $R_2$, $R_3$, $R_4$ et $R_5$ sont choisis parmi hydrogène, méthyle ou fluor.

**4)** Composé de formule (I) selon la revendication 1, dans lequel $X_1$ est fluor ou chlore.

**5)** Composé de formule (I) selon la revendication 1 dans lequel $R_1$ est isobutyle, 1,2-diméthylpropyle, 1,1,2-triméthylpropyle, 2,2-diméthylpropyle, 2-méthylprop-2-ényle ou (2-méthyl-1,3-dioxan-2-yl)méthyle.

**6)** Composé de formule (II):

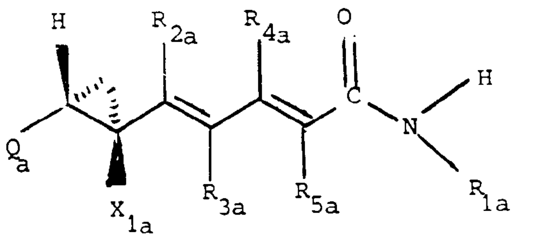

(II)

ou un sel de celui-ci, dans lequel $Q_a$ est un groupement phényle ou pyridyle substitué; $X_{1a}$ est hydrogène, fluor ou chlore; $R_{2a}$, $R_{3a}$, $R_{4a}$ et $R_{5a}$ sont identiques ou différents, l'un au moins étant hydrogène et les autres étant choisis indépendamment parmi hydrogène, halogéne, alkyle en $C_{1-4}$ ou halogénoalkyle en $C_{1-4}$; $X_{1a}$ est oxygène ou soufre; et $R_{1a}$ est choisi parmi hydrogène et hydrocarbyle en $C_{1-6}$ et hydrocarbyl en $C_{1-6}$ éventuellement substitué par dioxalanyle, halogéno, cyano, trifluorométhyle, trifluorométhylthio ou alkoxy en $C_{1-6}$.

**7)** Composé choisi parmi:

le (-)-(2E,4E)-N-(2-méthylprop-2-ényl)-5-[(1R,2S)-c-2-(3,4-dibromophényl)-r-1-fluorocyclopropyl]-3-méthylpenta-2,4-diénamide

le (-)-(2E/Z,4E)-N-(2-méthylprop-2-ényl)-5-[1R,2S)-c-2-(3,4-dibromophényl)-r-1-fluorocyclopropyl]-3-méthylpenta-2,4-diénamide

le (+)-(2E,4E)-N-(2-méthylprop-2-ényl)-5-[(1R,2R)-c-2-(3,4-dibromophényl)-r-1-fluorocyclopropyl]-3-méthyylpenta-2,4-diénamide

le (+)-(2E/Z,4E)-N-(2-méthylprop-2-ényl)-5-[(1S,2R)-c-2-(3,4-dibromophényl)-r-1-fluorocyclopropyl]-3-méthylpenta-2,4-diénamide

le (+)-(2E,4E)-N-isobutyl-5-[(1S,2R)-c-(3,4-dichlorophényl)-r-1-chlorocyclopropyl]-3-méthylpenta-2,4-diénamide

le (-)-(2E,4E)-N-isobutyl-5-[(1R,2S)-c-(3,4-dichlorophényl)-r-1-chlorocyclopropyl]-3-méthylpenta-2,4-diénamide

le (-)-(2E,4E)-N-(S-1-méthylpropyl)-5-[(1R,2S)-c-(3,4-dichlorophényl)-r-1-chlorocyclopropyl]-3-méthylpenta-2,4-diénamide

le (-)-(2E,4E)-N-(R-1-méthylpropyl)-5-[(1R,2S)-c-(3,4-dichlorophényl)-r-1-chlorocyclopropyl]-3-méthylpenta-2,4-diénamide

le (-)-(2E/Z,4E)-N-isobutyl-5-[(1S,2R)-trans-2-(3,4-dibromophényl)cyclopropyl]-3-méthylpenta-2,4-diénamide

le (+)-(2E/Z,4E)-N-Isobutyl-5-[(1R,2S)-trans-2-(3,4-dibromophényl)cyclopropyl-3-méthylpenta-2,4-diénamide

**8)** Procédé de préparation d'un composé selon la revendication 1 comprenant:

a) une réaction d'oxydation d'un alcool approprié de formule (III):

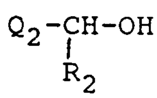

dans laquelle $R_2$ est tel que défini précédemment pour obtenir l'aldéhyde correspondant de formule ($III_A$):

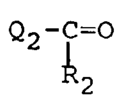

b) que l'on soumet par une réaction de type Wittig à un réactif capable d'introduire le groupement

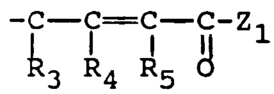

$R_3$, $R_4$, $R_5$ et $Z_1$ sont tels que définis précédemment puis à l'action d'une amine de formule $H_2NR_1$, dans laquelle $R_1$ est tel que défini précédemment et $Z_1$ est hydroxy, alkoxy en $C_{1-6}$, halogéno ou un ester de phosphoroimidate (-P(O)(O-aryl)NH- aryle où aryle est un aryle en $C_{6-10}$)

et ensuite la transformation éventuelle d'un composé de formule (I) en un autre composé de formule (I) par des méthodes bien connues de l'homme de l'art.

**9)** Variante du procédé selon la revendication 8, dans laquelle l'aldéhyde de formule ($III_A$) est préparé par hydrolyse acide d'un cétal, d'un ester énolique, d'un acétal ou par réduction du nitrile approprié.

**10)** Nouvel intermédiaire chimique de formule $Q_2CR_2=CR_3-CR_4=CR_5(=O)Z_1$, $Q_2COR_2$ ou

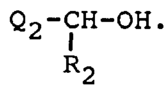

**11)** Composition insecticide ou acaricide comportant un composé de formule (I) tel que défini dans la revendication 1, en mélange avec un support ou un diluant.

**12)** Composition pesticide synergique comportant un composé de formule (I) tel que défini dans la revendication 1, un synergiste du composé de formule (I) et un support ou un diluant.

**13)** Utilisation des produits de formule (I) comme pesticides dans la lutte contre les organismes nuisibles à un environnement susceptible d'être infesté.

**14)** Utilisation selon la revendication 13, dans laquelle l'environnement est un animal.

**15)** Utilisation selon la revendication 13, dans laquelle l'environnement est une plante ou un arbre.

**16)** Utilisation selon la revendication 13, dans laquelle l'environnement est constitué de produits stockés.

**17)** Utilisation selon la revendication 13, des produits de formule (I) en association avec un ou plusieurs produits pesticides choisis parmi les pyréthrinoïdes et les carbamates.